(19) Europäisches Patentamt
European Patent Office
Office européen des brevets

(11) EP 1 100 777 B1

(12) EUROPEAN PATENT SPECIFICATION

(45) Date of publication and mention of the grant of the patent:
**28.05.2003 Bulletin 2003/22**

(21) Application number: **99936609.9**

(22) Date of filing: **21.07.1999**

(51) Int Cl.[7]: **C07D 207/34**, A61K 31/40

(86) International application number:
**PCT/EP99/05348**

(87) International publication number:
**WO 00/006541 (10.02.2000 Gazette 2000/06)**

# (54) SULFURATED DISTAMYCIN DERIVATIVES, PROCESS FOR PREPARING THEM, AND THEIR USE AS ANTITUMOR AGENTS

THIOETHERGRUPPEN ENTHALTENDE DISTAMYCIN-DERIVATE, VERFAHREN ZU IHRER HERSTELLUNG UND IHRE VERWENDUNG ALS ANTITUMORMITTEL

DERIVES DISTAMYCINE SULFURES, LEUR PROCEDE DE PREPARATION ET UTILISATION EN TANT QU'AGENTS ANTITUMORAUX

(84) Designated Contracting States:
**DE GB IT**

(30) Priority: **30.07.1998 GB 9816652**

(43) Date of publication of application:
**23.05.2001 Bulletin 2001/21**

(73) Proprietor: **Pharmacia Italia S.p.A.
20152 Milano (IT)**

(72) Inventors:
- **COZZI, Paolo
I-20133 Milan (IT)**
- **CALDARELLI, Marina
I-20147 Milan (IT)**
- **BERIA, Italo
I-45030 Villamarzana (IT)**
- **GERONI, Maria, Cristina
I-20149 Milan (IT)**
- **CAPOLONGO, Laura
I-20147 Milan (IT)**

(56) References cited:
**EP-A- 0 246 868**
**WO-A-94/20463**
**WO-A-97/03957**
**WO-A-97/28123**
**WO-A-97/43258**
**DD-A- 297 638**
**US-A- 5 395 849**

- **F. M. ARCAMONE ET AL.: JOURNAL OF MEDICINAL CHEMISTRY, vol. 32, no. 4, 1989, pages 774-8, XP000608784 cited in the application**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to new alkylating antitumor agents analogous to Distamycin A, to a process for their preparation, to pharmaceutical compositions containing them and to their use as therapeutic agents.

Distamycin A, whose formula is reported below

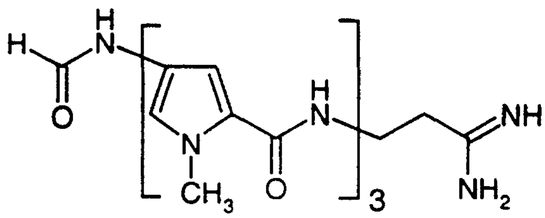

belongs to the family of the pyrroleamidine antibiotics and it is reported to interact reversibly and selectively with DNA-AT sequences, thus interfering with both replication and transcription. See, for a reference, Nature, 203, 1064 (1964); FEBS Letters, 7 (1970) 90; Prog. Nucleic Acids Res. Mol. Biol., 15, 285 (1975).

Several analogous to distamycin are known in the art.

DE-A-1795539 discloses distamycin derivatives in which the formyl group is replaced by a hydrogen atom or by the carboxylic acid residue of a $C_1$-$C_4$ aliphatic or cyclopentylpropionic acid.

EP-A-246,868 describes distamycin analogues in which the distamycin formyl group is substituted by aromatic, alicyclic or heterocyclic moieties bearing alkylating groups.

WO 97/28123 and WO 97/43258 describe distamycin analogues in which the amidino group is replaced with different nitrogen-containing ending groups and the distamycin formyl group is substituted by an aromatic or a cinnamoyl moiety, respectively.

It has now been found that a new class of distamycin derivatives as defined hereinunder, wherein the distamycin formyl group is substituted by a phenylcarbonyl, phenylalkylcarbonyl or phenylalkenylcarbonyl group bearing a haloethyl-thio group as an alkylating moiety, and the amidino group is optionally replaced by various nitrogen-containing ending groups, shows valuable biological properties.

Therefore, the present invention provides compounds which are sulfurated distamycin derivatives of formula:

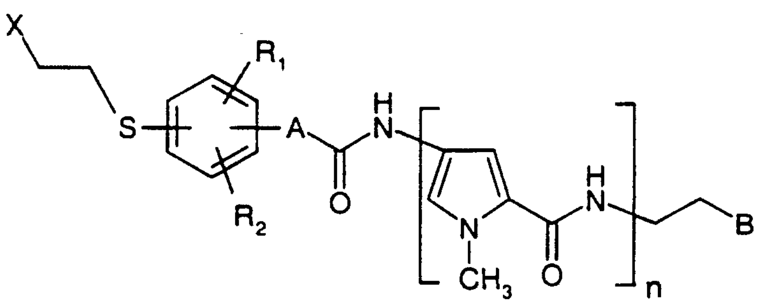

(I)

wherein:

n is 2, 3 or 4;
A is a bond, a $C_1$-$C_4$ alkylene or $C_2$-$C_4$ alkenylene group;
$R_1$ and $R_2$, which are the same or different, are selected from hydrogen, $C_1$-$C_4$ alkyl optionally substituted by one or more fluorine atoms, and $C_1$-$C_4$ alkoxy;
X is a halogen atom;
B is selected from:

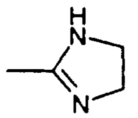, 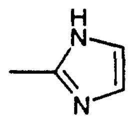, 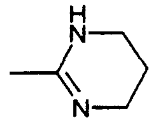, 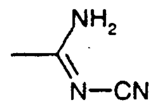, 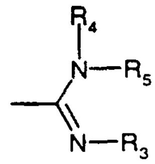, 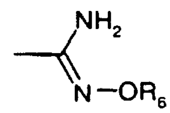,

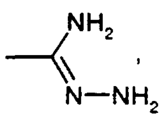, —CN, 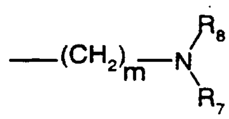, 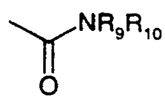 and 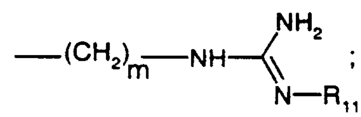;

wherein $R_3$, $R_4$, $R_5$, $R_6$, $R_7$, $R_8$, $R_9$ and $R_{10}$, which are the same or different, are selected from hydrogen or $C_1$-$C_4$ alkyl; $R_{11}$ is hydrogen, $C_1$-$C_4$ alkyl or hydroxy, and m is 0, 1 or 2; or pharmaceutically acceptable salts thereof.

The present invention includes within its scope also all the possible isomers covered by the compounds of formula (I), both separately and in admixture, as well as the metabolites and the pharmaceutically acceptable bioprecursors (otherwise known as pro-drugs) of the compounds of formula (I).

In the present description, unless otherwise specified, both terms alkyl and alkoxy include straight or branched $C_1$-C, alkyl and alkoxy groups such as, for instance, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy and tert-butoxy.

Preferred $C_1$-$C_4$ alkyl or alkoxy groups are methyl, ethyl, methoxy and ethoxy groups.

When substituted by one or more fluorine atoms, the $C_1$-$C_4$ alkyl groups are preferably $C_1$-$C_4$ perfluoroalkyl groups, e.g. trifluoromethyl.

Both terms alkylene and alkenylene refer, respectively, to $C_1$-$C_4$ alkylene or $C_2$-$C_4$ alkenylene groups, as bivalent radicals of the corresponding $C_1$-$C_4$ saturated or $C_2$-$C_4$ unsaturated hydrocarbons.

Preferred alkylene or alkenylene groups according to the present invention are methylene, ethylene or vinylene groups.

The term halogen atom includes fluorine, chlorine, bromine and iodine, being chlorine and bromine preferred.

Within the compounds of formula (I) the haloethyl-thio group and the A group are in ortho, meta or para position with respect to each other; preferably, the haloethyl-thio and A groups are in meta or para position.

Pharmaceutically acceptable salts of the compounds of formula (I) are their salts with pharmaceutically acceptable either inorganic or organic acids such as, for instance, hydrochloric, hydrobromic, sulfuric, nitric, acetic, propionic, succinic, malonic, citric, tartaric, methanesulfonic and p-toluenesulfonic acid.

A preferred class of compounds of the present invention is that wherein, in formula (I):

n is 3;
A is a bond or vinylene;
$R_1$ and $R_2$ which are the same or different, are selected from hydrogen, methyl, methoxy or trifluoromethyl;
X is chloro or bromo;
B is selected from:

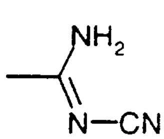, 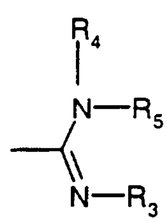, 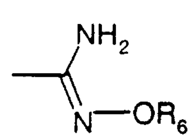, —CN,

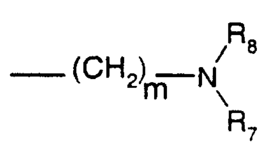, 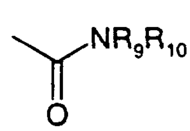 and 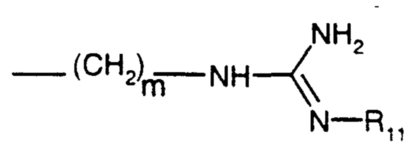

wherein $R_3$, $R_4$, $R_5$, $R_7$, $R_8$, $R_9$, $R_{10}$ and $R_{11}$, which are the same or different, are selected from hydrogen or methyl; $R_6$ is hydrogen; and m is 0 or 1; or the-pharmaceutically acceptable salts thereof.

Examples of specific compounds according to the present invention, especially in the form of salts, preferably with hydrochloric acid, are the following:

3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N',N'-trimethylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamide;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N-dimethylamine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propion-N-methylamidine;
3-[1-bromoethylthio)phenyl-1-carboxamido]pyrrole-2-carboxamido]pyrrole-2-carboxamido]pyrrole-2-carboxamido]propionamidoxime;
2-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionitrile;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionitrile; and
2-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]ethylguanidine.

A further object of the present invention is a process for preparing the compounds of formula (I), and the pharmaceutically acceptable salts thereof, which process comprises:

(a) when B is other than

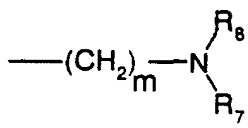

and

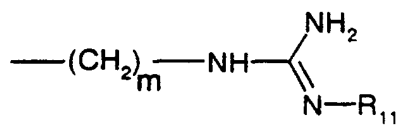

reacting a compound of formula:

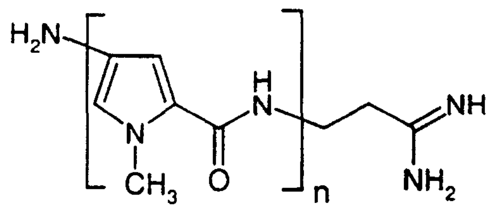

(II)

with a compound of formula:

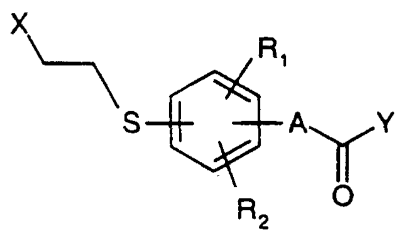

(III)

wherein n, $R_1$, $R_2$, X and A are as defined above, and Y is hydroxy or a suitable leaving group; so as to obtain a compound of formula:

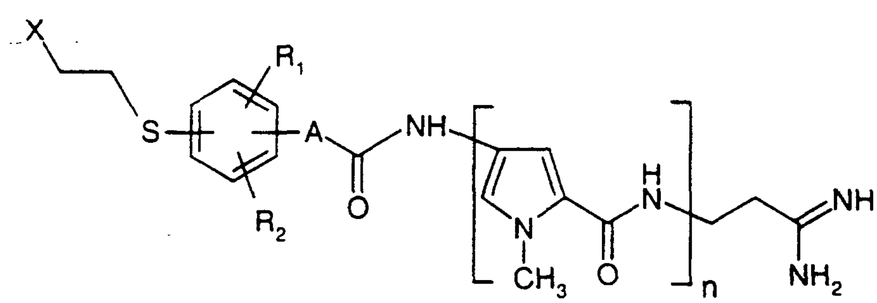

(Ia)

and, then, optionally reacting a compound of formula (Ia) with:

(i) $H_2N\text{-}(CH_2)_r\text{-}NH_2$, wherein r is 2 or 3, so as to obtain a compound of formula (I) having B equal to:

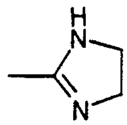 or 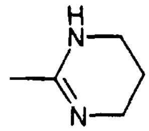

(ii) $H_2N-CH_2-CHO$, so obtaining a compound of formula (I) having B equal to:

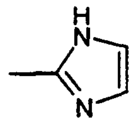

(iii)$H_2N-CN$, so obtaining a compound of formula (I) having B equal to:

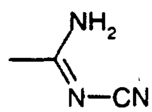

(iv) $H_2N-OR_6$, so obtaining a compound of formula (I) having B equal to:

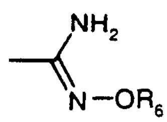

(v) $H_2N-NH_2$, so obtaining a compound of formula (I) having B equal to:

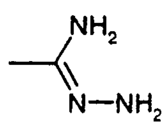

(vi) $HNR_4R_5$, so obtaining a compound of formula (I) having B equal to:

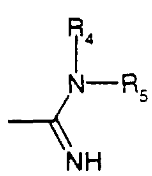

and then optionally with $H_2NR_3$, so obtaining a compound of formula (I) having B equal to:

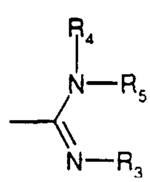

(vii)succinic anhydride, so obtaining a compound of formula (I) having B equal to -C≡N;
(viii)water in an alkaline medium, so obtaining a compound of formula (I) having B equal to $-CO-NR_9R_{10}$ wherein $R_9$ and $R_{10}$ are both hydrogen atoms;
(ix) $HNR_9R_{10}$, so obtaining a compound of formula (I) having B equal to:

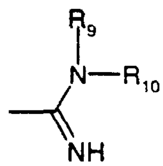

and then with water in an alkaline medium, so obtaining a compound of formula (I) having B equal to $-CO-NR_9R_{10}$, wherein $R_9$ and $R_{10}$ are, each independently, hydrogen or $C_1$-$C_4$ alkyl; or

(b) when B is other than

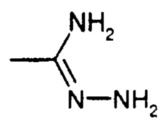

reacting a compound of formula:

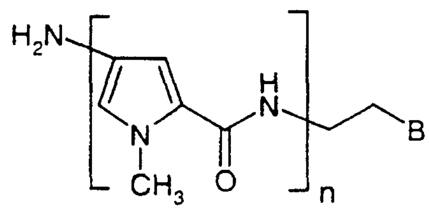
(IV)

with a compound of formula:

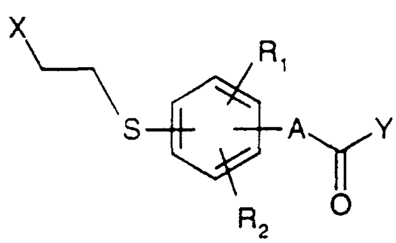
(III)

wherein n, B, $R_1$, $R_2$, X, Y and A are as defined above; so obtaining the corresponding compound of formula (I); and, if desired, converting the compound of formula (I) into a pharmaceutically acceptable salt thereof.

In formula (III), Y is hydroxy or a leaving group selected, for instance, from chloro, 2,4,5-trichlorophenoxy, 2,4-dinitro-phenoxy, succinimido-N-oxy, imidazolyl group, and the like.

The condensation reactions as set forth above under processes (a) and (b) is carried out according to known methods, for instance those described in the aforementioned EP-A-246,868.

The reaction between a compound of formula (II) or (IV) with a compound of formula (III) is preferably carried out with a molar ratio (II) : (III) or (IV) : (III) of from 1:1 to 1:2.

Within the compounds of formula (III) wherein Y is hydroxy, the reaction is carried out in an organic solvent, such as, dimethylsulphoxide, hexamethylphosphotriamide, dimethylacetamide, dimethylformamide, ethanol, phenyl, or pyridine, in the presence of an organic or inorganic base such as triethylamine, diisopropyl ethylamine, or sodium or potassium carbonate or bicarbonate, and of a condensing agent such as, N-ethyl-N'-(3-dimethylamino-propyl)-carbodiimide, N,N'-dicyclohexyl-carbodiimide, or 1-hydroxy-benzotriazole hydrate.

The reaction temperature may vary from about -10°C to about 100°C, and the reaction time from about 1 to about 24 hours.

Within the compounds of formula (III) wherein Y is a leaving group as set forth above, the aforementioned condensation reaction may be carried out in an organic solvent such as, for instance, dimethylformamide, dioxane, pyridine, tetrahydrofurane, or mixtures thereof with water, optionally in the presence of an organic or inorganic base, e.g. N,N'-diisopropylethylamine, triethylamine, sodium or potassium bicarbonate, at a temperature of from about 0°C to about 100°C, and for a time varying from about 2 hours to about 48 hours.

The reaction between a compound of formula (Ia) according to process (a) and one of the reactants as described above at points (i)-(vi) or (ix), can be carried out according to known methods, for instance those reported in US-4,766,142; WO 97-/28123; Chem. Revs. 1961, 155; J. Med. Chem. 1984, 27, 849-857; Chem. Revs. 1970, 151; and "The Chemistry of Amidines and Imidates", edited by S. Patai, John Wiley & Sons, N.Y. (1975).

The reaction of a compound of formula (Ia) with succinic anhydride, as defined in point (vii) above, is preferably carried out with a molar ratio (Ia):succinic anhydride of from 1:1 to 1:3 in an organic solvent such as, for instance, dimethyl sulphoxide or dimethylformamide, and in the presence of an organic or inorganic base such as, e.g., triethylamine, diisopropylethylamine, sodium or potassium carbonate, and the like. The reaction temperature may vary from about 25°C to about 100°C, and the reaction time from about 1 hour to about 12 hours.

The reaction with water in an alkaline medium, as defined in points (viii) and (ix) above, may be carried out according to known methods usually employed for alkaline hydrolysis, for instance by treating the substrate with an excess of sodium or potassium hydroxide in water or in a water/organic solvent admixture, e.g. dioxane, tetrahydrofuran, or acetonitrile, at a temperature of from about 50°C to about 100°C, for a time varying from about 2 hours to about 48 hours.

The compounds of formula (II) are known or may be prepared according to known methods; see, for a reference, Arcamone et al. in Gazzetta Chim. Ital. 97, 1097 (1967).

Also the compounds of formula (III) are known or may be prepared according to known methods, for instance by working as described in J. Org. Chem. 1993, 58, 4506-4508 or Helvetica Chimica Acta, Vol. 67, (1984), 1316-1327.

The compounds of formula (IV) are known compounds as well, for instance as reported in the aforementioned WO 97/28123.

In view of what above reported, it is clear to the man skilled in the art that when preparing the compounds of formula (I) as set forth above, optional amino groups, i.e.

$R_7$ and/or $R_8$ of the compounds of formula (IV) equal to hydrogen, need to be properly protected according to conventional techniques, so as to avoid unwanted side reactions.

Likewise, the conversion of the said protected amino groups into the free amines may be carried out according to known procedures. See, for a general reference, J. Org. Chem. 43, 2285, (1978); J. Org. Chem. 44, 811 (1979); J. Am. Chem. Soc. 78, 1359 (1956); Ber. 65, 1192 (1932); and J. Am Chem. Soc. 80, 1154, (1958).

Salification of a compound of formula (I), as well as preparation of a free compound starting from a salt, may be carried out by known standard methods.

Well known procedures such as, e.g., fractional crystallisation or chromatography, may also be followed for separating a mixture of isomers of formula (I) into the single isomers.

The compounds of formula (I) may be purified by conventional techniques such as, e.g., silica gel or alumina column chromatography, and/or by recrystallisation from an organic solvent such as, e.g., a lower aliphatic alcohol, e.g. methyl, ethyl or isopropyl alcohol, or dimethylformamide.

## PHARMACOLOGY

The compounds of formula (I) according to the present invention are useful as antineoplastic agents. Particularly, they show cytostatic properties towards tumor cells, so that they can be useful to inhibit growth of various tumors in mammals, including humans, such as, for instance, carcinomas, e.g. mammary carcinoma, lung carcinoma, bladder carcinoma, colon carcinoma, ovary and endometrial tumors. Other neoplasias in which the compounds of the present invention can find application are, for instance, sarcomas, e.g. soft tissue and bone sarcomas, and the hematological malignancies such as, e.g. leukemias.

The in vitro antitumor activity of the compounds of formula (I) was evaluated by cytotoxicity studies carried out on murine L1210 leukemia cells. Cells were derived from in vivo tumors and established in cell culture. The inhibition of cell growth was determined by counting surviving cells with a Coulter Counter after 48 hours treatment.

The in vitro activity was calculated on concentration-response curves and reported as $IC_{50}$ (concentration inhibiting 50% of the cellular growth in respect to controls) were calculated on dose-response.

The compounds of the invention were tested also in vivo on L1210 murine leukemia and on murine reticulosarcoma M 5076, showing a very good antitumoral activity, with the following procedure.

L1210 murine leukemia was maintained in vivo by i.p. weekly transplantation in CD2F1 female mice, obtained from Charles River Italy. For experiments, $10^5$ cells/mouse were injected i.v. in the same strain of mice. Animals were 8 to 10 weeks old at the beginning of the experiments. Compounds were administered i.v. at day +1 after tumor cells injections.

M5076 reticulosarcoma was maintained in vivo by i.m. serial transplantation. For experiments, $5x10^5$ cells/mice were injected i.m. in the same strain of mice. Animals were 8 to 10 weeks old at the beginning of the experiments. Compounds were administered i.v. at day 3, 7 and 11 after tumor injection.

Survival time of mice and tumor growth were calculated and activity was expressed in term of T/C% and T.I.%.

$$T/C = \frac{\text{median survival time treated group}}{\text{median survival time untreated group}} \times 100$$

T.I.= % inhibition of tumor growth respect to control

Tox = number of mice which died for toxicity.

Tox determination was made when mice died before the control and/or tested significant body weight loss and/or spleen and/or liver size reduction were observed.

The compounds of the invention can be administered to mammals, including humans, through the usual routes, for example, parenterally, e.g. by intravenous injection or infusion, intramuscularly, subcutaneously, topically or orally. The dosage depends on age, weight and conditions of the patient and on the administration route. For example, a suitable dosage for administration to adult humans may range from about 0.1 to about 150-200 mg pro dose 1-4 times a day.

Further object of the present invention are pharmaceutical compositions, which comprise a compound of formula (I) as an active principle, in association with one or more pharmaceutically acceptable carrier and/or diluent.

The pharmaceutical compositions of the present invention are usually prepared following conventional methods and are administered in a pharmaceutically suitable form. For instance, solutions for intravenous injection or infusion may contain as a carrier, for example, sterile water or preferably, they may be in the form of sterile aqueous isotonic saline solutions.

Suspensions or solutions for intramuscular injections may contain, together with the active compound a pharmaceutically acceptable carrier, e.g. sterile water, olive oil, ethyl oleate, glycols, e.g. propylene glycol, and if desired, a suitable amount of lidocaine hydrochloride.

In the forms for topical application, e.g. creams, lotions or pastes for use in dermatological treatment, the active ingredient may be mixed with conventional oleaginous or emulsifying excipients.

The solid oral forms, e.g. tablets and capsules, may contain, together with the active compound, diluents, e. g., lactose, dextrose, saccharose, cellulose, corn starch and potato starch; lubricants, e.g. silica, talc, stearic acid, magnesium or calcium stearate, and/or polyethylene glycols; binding agents, e.g. starches, arabic gums, gelatin, methylcellulose, carboxymethyl cellulose, polyvinylpyrrolidone; disaggregating agents, e.g. starch, alginic acid, alginates, sodium starch glycolate; effervescing mixtures; dyestuffs; sweeteners; wetting agents, for instance, lecithin, polysorbates, laurylsulphates; and, in general, non-toxic and pharmacologically inactive substances used in pharmaceutical formulation. Said pharmaceutical preparations may be manufactured by known techniques, for example by means of mixing, granulating, tabletting, sugar-coating or film-coating processes.

Further object of the present invention are the compounds of formula (I) for use in a method for treating the human or animal body by therapy.

Furthermore, the present invention provides a method for treating tumors in a patient in need of it, which comprises administering to said patient a composition of the invention.

A further object of the present invention is a combined method for treating cancer or for ameliorating the conditions of mammals, including humans, suffering from cancer, said method comprising administering a compound of formula (I), or a pharmaceutically acceptable salt thereof, and an additional antitumor agent, close enough in time and in amounts sufficient to produce a therapeutically useful effect.

The present invention also provides products containing a compound of formula (I), or a pharmaceutically acceptable salt thereof, and an additional antitumour agent as a combined preparation for simultaneous, separate or sequential use in anti-cancer therapy.

The term "antitumor agent" is meant to comprise both a single antitumor drug and "cocktails" i.e. a mixture of such drugs, according to the clinical practice. Examples of antitumor agents that can be formulated with a compound of formula (I), or alternatively, can be administered in a combined method of treatment, include doxorubicin, daunomycin, epirubicin, idarubicin, etoposide, fluorouracil, melphalan, cyclophosphamide, 4-demethoxy daunorubicin, bleomycin, vinblastin, and mitomycin, or mixtures thereof.

The following examples are given to better illustrate the present invention but do not limit the scope of the invention itself.

## Example 1

**3-[1-methyl-4[1-methyl-4[1-methyl-4[4-(2-chloroethylthio)phenyl-1-carboxamido]pyrrole-2-carboxamido] pyrrole-2-carboxamido]pyrrole-2-carboxamido]propionamidine**

***Step I:*** **The intermediate 4-(2-hydroxyethyl)thiobenzoic acid**

To a solution of 400 mg of 4-thiobenzoic acid in 2.85 ml of NaOH 2N, 0.160 ml of 2-chloroethanol were added. The solution was refluxed for 1 hour, 2.85 ml of hydrochloric acid 2N were then added dropwise and the precipitated was filtered and dried giving 370 mg of a white solid.
FAB-MS: m/z 220, (60, $[M+H]^+$)
PMR ($CDCl_3$) d:
7.61 (d, J= 15.7 Hz, 1H), 7.33 (m, 2H), 6.55 (m, 2H), 6.21 (d, J= 15.7 Hz, 1H), 4.22 (q, J=7.1 Hz, 2H), 3.9 (b.s., 1H), 3.19 (q, J=7.1 Hz, 2H), 1.25 (t, J=7.1 Hz, 3H), 1.28 (t, J=7.1 Hz, 3H).

By analogous procedures and by using the opportune starting materials the following intermediate compounds can be obtained:
3-methyl-4(2-hydroxyethyl)thiobenzoic acid; 4-(2-hydroxyethyl)thiocinnamic acid
FAB-MS: m/z 224
PMR (DMSO-$d_6$) d:
7.59 (m, 2H), 7.52 (d, J = 16.0 Hz; 1H), 7.31 (m, 2H), 6.46 (d, J= 16.0 Hz, 1H), 4.9 (bs, 1H), 3.57 (t, J=6.8 Hz, 2H), 3.08 (t, J=6.8 Hz, 2H).

***Step II:*** **The title compound**

A solution of 240 mg of the intermediate, as prepared in step I, and 0.7 ml of thionyl chloride in 10 ml of toluene were refluxed for four hours, then the solvent was evaporated in vacuo. The crude residue was dissolved in 20 ml toluene and added portionwise to a solution of 500 mg of 3-[1-methyl-4-[1-methyl-4-[1-methyl-4-aminopyrrole-2-carboxamido]pyrrole-2-carboxamido]pyrrole-2-carboxamido]propionamidine dihydrochloride (prepared as reported in J. Med. Chem 32, 774-778, 1989) and 160 mg of potassium bicarbonate in 10 ml of water.

The mixture was vigorously stirred at room temperature for one hour, the solvent was evaporated in vacuo and the crude residue purified by flash chromatography (methylene chloride/ methanol: 85/15) to yield 350 mg of the title compound as a white solid.
FAB-MS: m/z 652, (100, $[M+H]^+$)
PMR (DMSO-$d_6$) d:
10.34 (s, 1H), 9.98 (s, 1H), 9.92 (s, 1H), 8.9 (b.s., 2H), 8.6 (b.s., 2H), 8.21 (t, J=5.6 Hz, 1H), 7.91 (m, 2H), 7.47 (m, 2H), 7.32 (d, J=1.7 Hz, 1H), 7.24 (d, J=1.7 Hz, 1H), 7.18 (d, J=1.7 Hz, 1H), 7.10 (d, J=1.7 Hz, 1H), 7.06 (d, J=1.7 Hz, 1H), 6.95 (d, J=1.7 Hz, 1H), 3.86 (s, 3H), 3.83 (s, 3H), 3.80 (s, 3H), 3.78 (t, J=7.3 Hz, 2H), 3.48 (m, 4H), 2.60 (t, J=6.5 Hz, 2H).

By analogous procedures and by using the opportune starting materials the following compounds can be obtained:

3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N',N'-trimethylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamide;
2-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N-dimethylamine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[3-methyl-4-(2-chloroethylthio)phenyl-l-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propion-N-methylamidine;
2-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionitrile;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidoxime;
2-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]ethylguanidine.

## Example 2

### 9) 3-[1-methyl-4[1-methyl-4[1-methyl-4[4-(2-chloroethylthio)phenyl-1-carboxamido]pyrrole-2-carboxamido] pyrrole-2-carboxamido]pyrrole-2-carboxamido]propionitrile

To a solution of 200 mg of 3-[1-methyl-4[1-methyl-4[1-methyl-4[4-(2-chloroethylthio)phenyl-1-carboxamido] pyrrole-2-carboxamido]pyrrole-2-carboxamido]pyrrole-2-carboxamido]propionamidine hydrochloride (prepared as reported in example 1) in 10 ml DMF were added 60 mg of potassium carbonate and 35 mg of succinic anhydride. The mixture was heated at 60°C for 2 hours. The solvent evaporated under vacuum and the crude residue purified by flash chromatography (methylene chloride/methanol : 8/2) to yield 120 mg of the title compound as a white powder.
FAB-MS: m/z 635, (100, $[M+H]^+$)
PMR (DMSO-$d_6$) d:
10.30 (s, 1H), 9.96 (s, 1H), 9.91 (s, 1H), 8.31 (t, J=5.7 Hz, 1H), 7.90 (m, 2H), 7.48 (m, 2H), 7.32 (d, J=1.7 Hz, 1H), 7.24 (d, J=1.7 Hz, 1H), 7.20 (d, J=1.7 Hz, 1H), 7.08 (d, J=1.7 Hz, 1H), 7.05 (d, J=1.7 Hz, 1H), 6.93 (d, J=1.7 Hz, 1H), 3.86 (s, 3H), 3.84 (s, 3H), 3.80 (s, 3H), 3.78 (t, J=7.0 Hz, 2H), 3.44 (m, 4H), 2.72 (t, J=6.5 Hz, 2H).

By analogous procedure and by using the opportune starting materials the following compounds can be obtained:

3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N'-dimethylamidine;
FAB-MS: m/z 680, (100, $[M+H]^+$)
PMR (DMSO-$d_6$) d:
10.32 (s, 1H), 9.96 (s, 1H), 9.91 (s, 1H), 9.0 (b.s., 2H), 8.21 (t, J=5.6 Hz, 1H), 7.91 (m, 2H), 7.47 (m, 2H), 7.31 (d, J=1.7 Hz, 1H), 7.23 (d, J=1.7 Hz, 1H), 7.18 (d, J=1.7 Hz, 1H), 7.10 (d, J=1.7 Hz, 1H), 7.06 (d, J=1.7 Hz, 1H), 6.93 (d, J=1.7 Hz, 1H), 3.86 (s, 3H), 3.83 (s, 3H), 3.79 (s, 3H), 3.44 (m, 4H), 3.00 (s, 3H), 2.77 (s, 3H), 2.71 (t, J=6.8 Hz, 2H).
3-[1-methyl-4[1-methyl-4[1-methyl-4[4-(2-chloroethylthio)phenyl-l-carboxamido]pyrrole-2-carboxamido]pyrrole-2-carboxamido]pyrrole-2-carboxamido]propion-N-methylamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)phenyl-l-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N-dimethylamine;
3-[1-bromoethylthio)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[3-methyl-4-(2-chloroethylthio)phenyl-l-carboxamido]pyrrole-2-carboxamido] pyrrole-2-carboxamido]pyrrole-2-carboxamido]propionamidoxime;

3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]ethylguanidine.

## Example 3

**14) 3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidine**

***Step I:*** **The intermediate 4-(2-chloroethyl)thiocinnamic acid**

To a solution of 150 mg of 4-(2-hydroxyethyl)thiocinnamic acid (prepared as reported in example 1 step I) in 3 ml of pyridine, 0.105 ml of mesyl chloride were added and the solution was warmed for 2 hours at 80°C. The solution was cooled at room temperature and hydrochloric acid 37% was slowly added until pH=1. The precipitate obtained was filtered and washes with water then dried obtaining 100 mg of orange solid.
FAB-MS: m/z 242
PMR (DMSO-$d_6$) d:
12.3 (bs, 1H); 7.63 (m, 2H); 7.54 (d, J = 15.9 Hz, 1H); 7.34 (m, 2H); 6.48 (d, J = 15.9 Hz, 1H); 3.76 (t, J = 7.1 Hz, 2H); 3.40 (t, J = 7.1 Hz, 2H).
By analogous procedure and by using the opportune starting materials the following products can be obtained:

4-(2-chloroethyl)thiobenzoic acid;
FAB-MS: m/z 216
PMR ($CDCl_3$) d:
8.01 (m, 2H); 7.38 (m, 2H); 3.67 (d, J = 7.0 Hz, 2H); 3.35 (d, J = 7.0 Hz, 2H).
4-(2-bromoethyl)thiobenzoic acid;
3-methyl-4-(2-chloroethyl)thiobenzoic acid.

***Step II:*** **The title compound**

A solution of 95 mg of 4-(2-chloroethyl)thiocinnamic acid (prepared as described in step I), 80 mg of dicyclohexylcarbodiimide and 53 mg of 1-hydroxybenzotriazole hydrate in 5 ml of DMF was stirred at 80°C for four hours, cooled at room temperature and then added with 200 mg 3-[1-methyl-4-[1-methyl-4-[1-methyl-4-aminopyrrole-2-carboxamido]pyrrole-2-carboxamido]pyrrole-2-carboxamido]propionamidine dihydrochloride (prepared as reported in J. Med.Chem 32,774-778,1989) and 58 mg of potassium bicarbonate.
The mixture was stirred at room temperature for 2 hours, the solvent was evaporated in vacuum and the crude residue purified by flash chromatography (methylene chloride/methanol: 8/2) to yield 130 mg of the title compound as a yellow solid.
FAB-MS: m/z 678, (100, $[M+H]^+$)
PMR (DMSO-$d_6$) d:
10.23 (s, 1H), 9.96 (s, 1H), 9.91 (s, 1H), 8.9 (b.s., 2H), 8.6 (b.s., 2H), 8.21 (t, J=5.6 Hz, 1H), 7.55 (m, 2H), 7.46 (d, J=75.8 Hz, 1H), 7.41 (m, 2H), 7.30 (d, J=1.7 Hz, 1H), 7.23 (d, J=1.7 Hz, 1H), 7.17 (d, J=1.7 Hz, 1H), 7.01 (d, J=1.7 Hz, 1H), 6.96 (d, J=1.7 Hz, 1H), 6.95 (d, J=1.7 Hz, 1H), 6.77 (d, J=15.8 Hz, 1H), 3.85 (s, 3H), 3.83 (s, 3H), 3.80 (s, 3H), 3.76 (t, J=7.3 Hz, 2H), 3.49 (m, 2H), 3.40 (t, J=7.3 Hz, 2H), 2.60 (t, J=6.4 Hz, 2H).
By analogous procedure and using the opportune starting material the following product can be obtained:

3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]ethylguanidine.

## Example 4

Tablets each weighing 0.250 g and containing 50 mg of the active substance can be manufactured as follows:

| *Composition for 10,000 tablets* | |
|---|---|
| 3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidine hydrochloride Lactose | 500 g<br>1,400 g |
| Corn starch | 500 g |
| Talc powder | 80 g |
| Magnesium stearate | 20 g |

3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidine hydrochloride, lactose and half of the corn starch were mixed; the mixture was then forced through a sieve of 0.5 mm mesh size.

Corn starch (10 g) was suspended in warm water (90 ml) and the resulting paste was used to granulate the powder. The granulate was dried, comminuted on a sieve of 1.4 mm mesh size, then the remaining quantity of starch, talc and magnesium stearate was added, carefully mixed and processed into tablets.

## Example 5

Capsules, each dosed at 0.200 g and containing 20 mg of the active substance can be prepared as follows:

| *Composition for 500 capsules* | |
|---|---|
| 3-[1-methyl-4[1-methyl-4[1-methyl-4[4-(2-chloroethylthio)phenyl-l-carboxamido]pyrrole-2-carboxamido]pyrrole-2-carboxamido]pyrrole-2-carboxamido]propionamidine hydrochloride Lactose | 10 g<br>80 g |
| Corn starch | 5 g |
| Magnesium stearate | 5 g |

This formulation can be encapsulated in two-piece hard gelatin capsules and dosed at 0.200 g for each capsule.

## Example 6

Intramuscular Injection 25 mg/ml

An injectable pharmaceutical composition can be manufactured by dissolving 25 g of 3-[1-methyl-4[1-methyl-4[1-methyl-4[4-(2-chloroethylthio)phenyl-l-carboxamido]pyrrole-2-carboxamido]pyrrole-2-carboxamido]pyrrole-2-carboxamido]propionamidine hydrochloride in sterile propyleneglycol (1000 ml) and sealing ampoules of 1-5 ml.

## Claims

1. A compound which is a sulfurated distamycin derivative of formula:

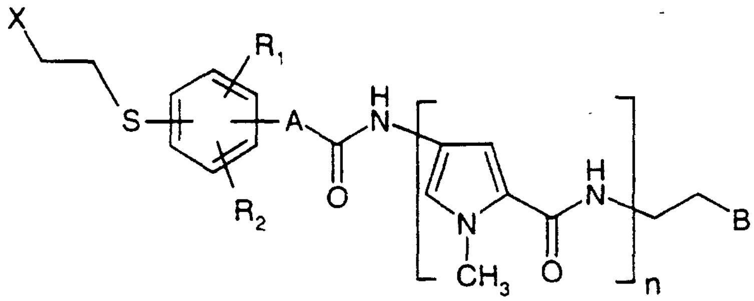

(I)

wherein:

n is 2, 3 or 4;
A is a bond, a $C_1$-$C_4$ alkylene or $C_2$-$C_4$ alkenylene group;
$R_1$ and $R_2$, which are the same or different, are selected from hydrogen, $C_1$-$C_4$ alkyl optionally substituted by one or more fluorine atoms, and $C_1$-$C_4$ alkoxy;
X is a halogen atom;
B is selected from:

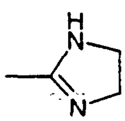 , 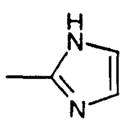 , 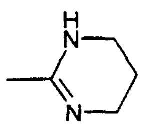 , 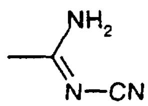 , 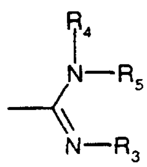 , 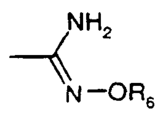 ,

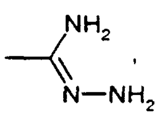 , —CN , 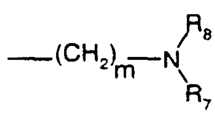 , 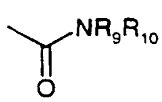 and 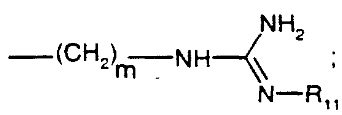 ;

wherein $R_3$, $R_4$, $R_5$, $R_6$, $R_7$, $R_8$, $R_9$ and $R_{10}$, which are the same or different, are selected from hydrogen or $C_1$-$C_4$ alkyl; $R_{11}$ is hydrogen, $C_1$-$C_4$ alkyl or hydroxy, and m is 0, 1 or 2; or a pharmaceutically acceptable salt thereof.

2. A compound according to claim 1 wherein $R_3$, $R_4$, $R_5$, $R_6$, $R_7$, $R_8$, $R_9$, $R_{10}$ and $R_{11}$ are, independently from each other, hydrogen, methyl or ethyl.

3. A compound accordi-ng to claim 1 or 2 wherein n is 3;
A is a bond or vinylene;
$R_1$ and $R_2$, which are the same or different, are selected from hydrogen, methyl, methoxy or trifluoromethyl;
X is chloro or bromo;
B is selected from:

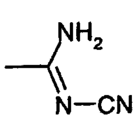 , 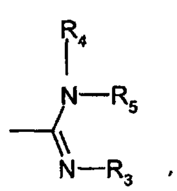 , 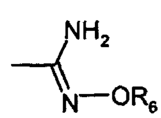 , —CN ,

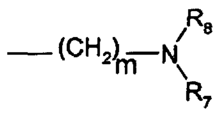 , 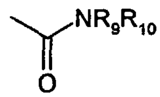 and 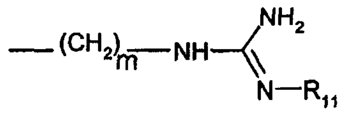

wherein $R_3$, $R_4$, $R_5$, $R_7$, $R_8$, $R_9$, $R_{10}$ and $R_{11}$, which are the same or different, are selected from hydrogen or methyl; $R_6$ is hydrogen; and m is 0 or 1; or a pharmaceutically acceptable salt thereof.

**4.** A compound according to claim 1 selected from the group consisting of:

3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamidine;
3-[1-methyl-4[1-methyl-4[1-methyl-4 [4- (2-chloroethylthio)phenyl-1-carboxamido]pyrrole-2-carboxamido]pyrrole-2-carboxamido]pyrrole-2-carboxamidol]propion-N,N'-dimethylamidine;
3- [1-methyl-4[1-methyl-4 [1-methyl-4[4- (2-chloroethylthio)phenyl-1-carboxamido]pyrrole-2-carboxamido] pyrrole-2-carboxamido]pyrrole-2-carboxamido]propion-N,N',N'-trimethylamidine;
3- [1-methyl-4[1-methyl-4[1-methyl-4 [4- (2-chloroethylthio)phenyl-1-carboxamido]pyrrole-2-carboxamido] pyrrole-2-carboxamido]pyrrole-2-carboxamido]propion-N-cyanamidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N-methylamide;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[4-(2-chloroethylthio)phenyl-1-carboxamido]pyrrole-2-carboxamido]pyrrole-2-carboxamido]pyrrole-2-carboxamido]propion-N,N-dimethylamine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamidine;
3-[1-methyl-4[1-methyl-4[1-methyl-4[4-(2-chloroethylthio)cinnamoyl-1-carboxamido]pyrrole-2-carboxamido] pyrrole-2-carboxamido]pyrrole-2-carboxamido]propionamidine;
3-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]propion-N-methylamidine;
3-[1-bromoethylthio)phenyl-1-carboxamido]pyrrole-2-carboxamido]pyrrole-2-carboxamido]pyrrole-2-carboxamido]propionamidoxime;
2-[1-methyl-4[1-methyl-4[1-methyl-4[4-(2-bromoethylthio)phenyl-1-carboxamido]pyrrole-2-carboxamido]pyrrole-2-carboxamido]pyrrole-2-carboxamido]ethylguanidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propion-N,N'-dimethylamidine;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamidoxime;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionamide;
3-[1-methyl-4[1-methyl-4[1-methyl-4[3-methyl-4-(2-chloroethylthio)phenyl-1-carboxamido]pyrrole-2-carboxamido]pyrrole-2-carboxamido]pyrrole-2-carboxamido]propionitrile;
3-[1-methyl-4[1-methyl-4[1-methyl-4[4-(2-chloroethylthio)cinnamoyl-1-carboxamido]pyrrole-2-carboxamido] pyrrole-2-carboxamido]pyrrole-2-carboxamido]propion-N-methylamidine;
3-[1-methyl-4[1-methyl-4[1-methyl-4[4-(2-chloroethylthio)cinnamoyl-1-carboxamido]pyrrole-2-carboxamido] pyrrole-2-carboxamido]pyrrole-2-carboxamido]propion-N,N'-dimethylamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propion-N-cyanamidine;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamidoxime;
3-[1-methyl-4[1-methyl-4[1-methyl-4[4-(2-chloroethylthio)cinnamoyl-1-carboxamido]pyrrole-2-carboxamido]pyrrole-2-carboxamido]pyrrole-2-carboxamido]propionamide;
3-[1-methyl-4[1-methyl-4[1-methyl-4[4-(2-chloroethylthio)cinnamoyl-l-carboxamido]pyrrole-2-carboxamido]pyrrole-2-carboxamido]pyrrole-2-carboxamido]propionitrile;
2-[1-methyl-4[1-methyl-4[1-methyl-4[4-(2-chloroethylthio)cinnamoyl-l-carboxamido]pyrrole-2-carboxamido]pyrrole-2-carboxamido]pyrrole-2-carboxamido]ethylguanidine;

and the pharmaceutically acceptable salts thereof.

**5.** A process for preparing a compound of formula (I) as defined in claim 1, which process comprises:

(a) when B is other than

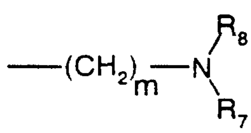

and

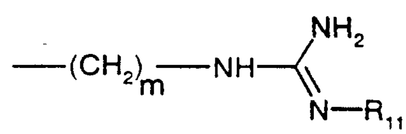

reacting a compound of formula:

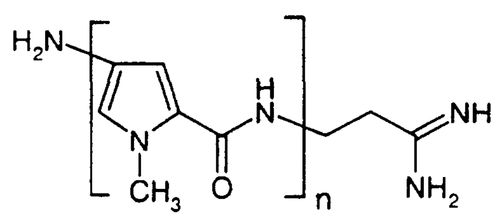

(II)

with a compound of formula:

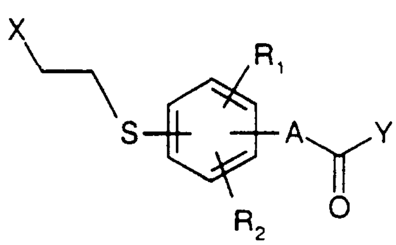

(III)

wherein n, $R_1$, $R_2$, X and A are as defined in claim 1, and Y is hydroxy or a suitable leaving group; so as to obtain a compound of formula:

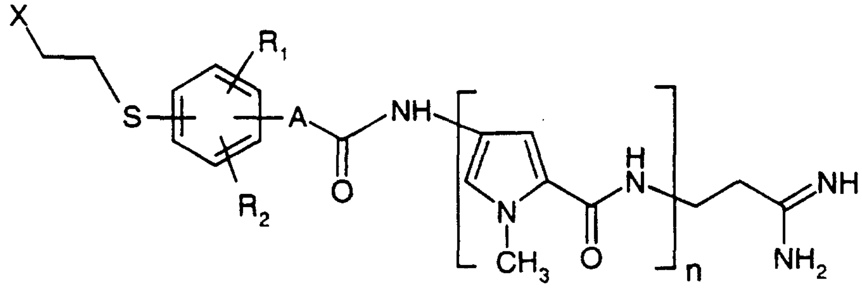

(Ia)

and, then, optionally reacting a compound of formula (Ia) with:

(i) $H_2N-(CH_2)_r-NH_2$, where r is 2 or 3, so as to obtain a compound of formula (I) having B equal to:

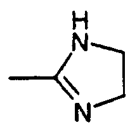
or
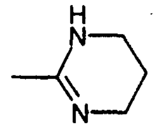

(ii) $H_2N-CH_2-CHO$, so obtaining a compound of formula (I) having B equal to:

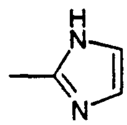

(iii)$H_2N-CN$, so obtaining a compound of formula (I) having B equal to:

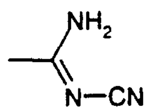

(iv) $H_2N-OR_6$, so obtaining a compound of formula (I) having B equal to:

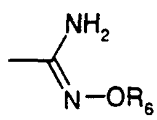

(v) $H_2N-NH_2$, so obtaining a compound of formula (I) having B equal to:

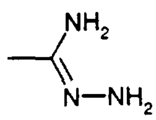

(vi) $HNR_4R_5$, so obtaining a compound of formula (I) having B equal to:

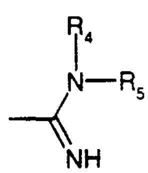

and then optionally with $H_2NR_3$, so obtaining a compound of formula (I) having B equal to:

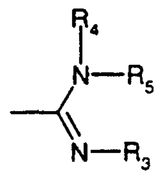

(vii)succinic anhydride, so obtaining a compound of formula (I) having B equal to -C≡N;
(viii)water in an alkaline medium, so obtaining a compound of formula (I) having B equal to $-CO-NR_9R_{10}$ wherein $R_9$ and $R_{10}$ are both hydrogen atoms;
(ix) $HNR_9R_{10}$, so obtaining a compound of formula (I) having B equal to:

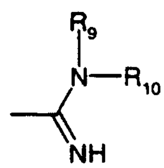

and then with water in an alkaline medium, so obtaining a compound of formula (I) having B equal to $-CO-NR_9R_{10}$, wherein $R_9$ and $R_{10}$ are as defined in claim 1; or:

(b) when B is other than

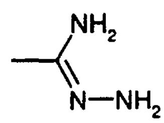

reacting a compound of formula:

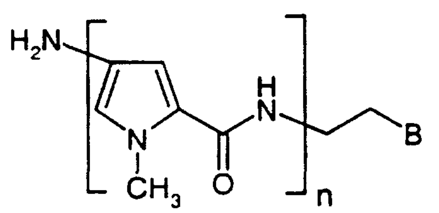

(IV)

with a compound of formula:

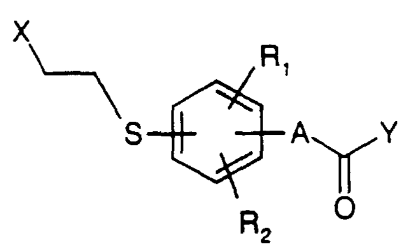

(III)

wherein n, B, $R_1$, $R_2$, X, Y and A are as defined above; so obtaining the corresponding compound of formula (I) and, if desired, converting the compound of formula (I) into a pharmaceutically acceptable salt thereof.

**6.** A process according to claim 5 wherein, in the compounds of formula (III), Y is hydroxy or a group selected from

chloro, 2,4,5-trichlorophenoxy, 2,4-dinitro-phenoxy, succinimido-N-oxy and imidazolyl.

**7.** A pharmaceutical composition comprising one or more pharmaceutically acceptable carriers and/or diluents and, as the active principle, a compound as defined in claim 1.

**8.** A compound as defined in claim 1 for use in a method of treatment of the human or animal body by therapy.

**9.** A compound as defined in claim 8 for use as an antitumor agent.

**10.** Use of a compound as defined in claim 1 in the manufacture of a medicament for use as an antitumor agent.

**Patentansprüche**

**1.** Sulfurierte Distamycinderivat-Verbindung der Formel:

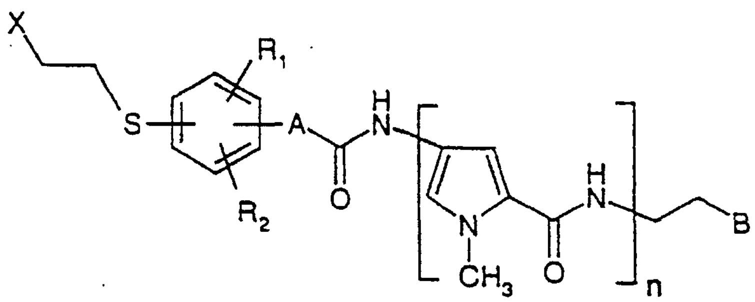

(I)

worin
n 2, 3 oder 4 ist;
A ist eine Bindung, eine $C_1$-$C_4$-Alkylen- oder $C_2$-$C_4$-Alkenylengruppe;
R1 und R2, die gleich oder verschieden sein können, sind ausgewählt aus Wasserstoff, C1-C4-Alkyl, gegebenenfalls substituiert mit einem oder mehreren Fluoratomen, und C1-C4-Alkoxy;
X ist ein Halogenatom;
B ist ausgewählt aus:

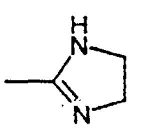 , 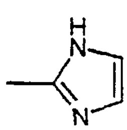 , 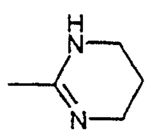 , 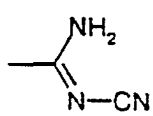 , 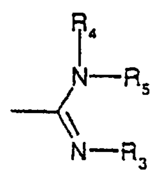 , 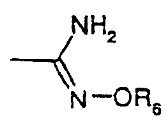 ,

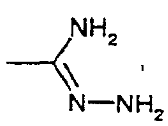 , —CN , 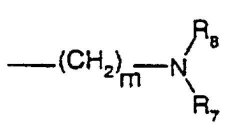 , 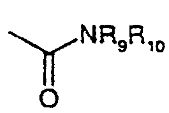 und 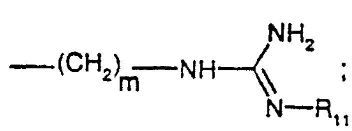 ;

wobei R3, R4, R5, R6, R7, R8, R9 und R10, die gleich oder verschieden sein können, ausgewählt sind aus Wasserstoff oder C1-C4-Alkyl; $R_{11}$ ist Wasserstoff, C1-C4-Alkyl oder Hydroxy, und m ist 0, 1 oder 2;
oder ein pharmazeutisch annehmbares Salz davon.

**2.** Verbindung gemäß Anspruch 1, wobei R3, R4, R5, R6, R7, R8, R9, R10 und R11 unabhängig voneinander Wasserstoff, Methyl oder Ethyl sind.

**3.** Verbindung gemäß Anspruch 1 oder 2, wobei n 3 ist;
A ist eine Bindung oder Vinylen;
R1 und R2, die gleich oder verschieden sein können, sind ausgewählt aus Wasserstoff, Methyl, Methoxy oder Trifluormethyl;
X ist Chlor oder Brom;
B ist ausgewählt aus:

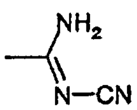 , 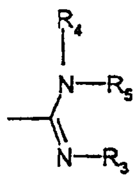 , 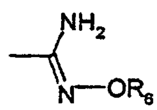 , —CN ,

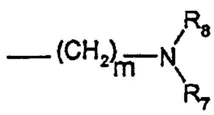 , 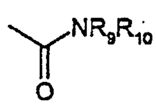 und 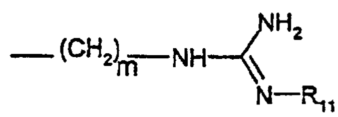

worin R3, R4, R5, R7, R8, R9, R10 und R11, die gleich oder verschieden sein können, ausgewählt sind aus Wasserstoff oder Methyl; R6 ist Wasserstoff; und m ist 0 oder 1;
oder ein pharmazeutisch annehmbares Salz davon.

**4.** Verwendung gemäß Anspruch 1, ausgewählt aus der Gruppe bestehend aus:

3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N,N',N'-trimethylamidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N-methylamid;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]ethylguanidin;
3-[1-Methyl-4[1-methyl-4[1-methyl-4[4-(2-chlorethylthio)phenyl-1-carboxamido]pyrrol-2-carboxamido]pyrrol-2-carboxamido]pyrrol-2-carboxamido]propion-N,N-dimethylamin;
3- [1-Methyl-4[1-methyl-4[1-methyl-4[4-(2-bromethylthio)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[3-methyl-4-(2-chlorethylthio)phenyl-1-carboxamido]pyrrol-2-carboxamido]pyrrol-2-carboxamido]pyrrol-2-carboxamido]propionamidin;
3-[1-Methyl-4[1-methyl-4[1-methyl-4[4-(2-chlorethylthio)cinnamoyl-1-carboxamido]pyrrol-2-carboxamido] pyrrol-2-carboxamido]pyrrol-2-carboxamido]propionamidin;
3-[1-Methyl-4[1-methyl-4[1-methyl-4[4-(2-bromethylthio)phenyl-1-carboxamido]pyrrol-2-carboxamido]pyrrol-2-carboxamido]pyrrol-2-carboxamido]propion-N-methylamidin;
3-[1-Bromethylthio)phenyl-1-carboxamido]pyrrol-2-carboxamido]pyrrol-2-carboxamido]pyrrol-2-carboxamido]propionamidoxim;
2-[1-Methyl-4[1-methyl-4[1-methyl-4[4-(2-bromethylthio)phenyl-1-carboxamido]pyrrol-2-carboxamido]pyrrol-2-carboxamido]pyrrol-2-carboxamido]ethylguanidin;
3-[1-Methyl-4[1-methyl-4[1-methyl-4[3-methyl-4-(2-chlorethylthio)phenyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[3-methyl-4-(2-chlorethylthio)phenyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[3-methyl-4-(2-chlorethylthio)phenyl-1-carboxamido]pyrrol-2-carboxamido]pyrrol-2-carboxamido]pyrrol-2-carboxamido]propionamid;
3-[1-Methyl-4[1-methyl-4[1-methyl-4[3-methyl-4 (2-chlorethylthio)phenyl-1-carboxamido]pyrrol-2-carboxamido]pyrrol-2-carboxamido]pyrrol-2-carboxamido]propionitril;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propion-N-methylamidin;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propion-N,N'-dimethylamidin;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propion-N-cyanamidin;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propionamidoxim;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propionamid;
3-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]propionitril;
2-[1-Methyl-4[1-methyl-4[1-methyl-4[4-(2-chlorethylthio)cinnamoyl-1-carboxamido]pyrrol-2-carboxamido]pyrrol-2-carboxamido]pyrrol-2-carboxamido]ethylguanidin;

oder den pharmazeutisch annehmbaren Salzen davon.

**5.** Verfahren zur Herstellung einer Verbindung der Formel (I), wie in Anspruch 1 definiert, wobei das Verfahren umfaßt:

(a) wenn B nicht

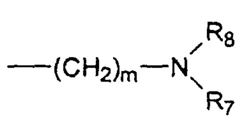 und 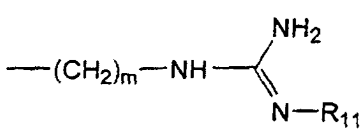

ist, Reagieren einer Verbindung der Formel:

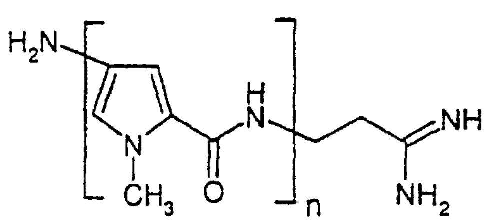 (II)

mit einer Verbindung der Formel:

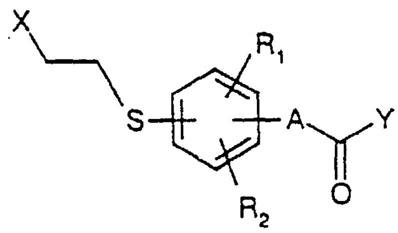

(III)

worin n, $R_1$ , $R_2$, X und A wie in Anspruch 1 definiert sind, und Y ist Hydroxy oder eine geeignete Abgangsgruppe;
um eine Verbindung der Formel

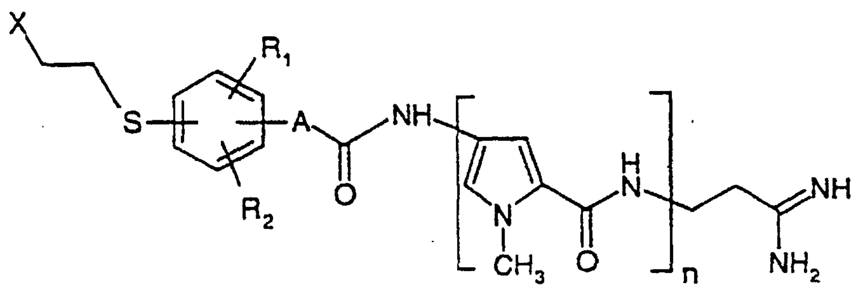

(Ia)

zu erhalten, und, anschließend, gegebenenfalls Reagieren einer Verbindung der Formel (Ia) mit:

(i) $H_2N-(CH_2)_r-NH_2$, wobei r 2 oder 3 ist, um eine Verbindung der Formel (I) zu erhalten, wobei B ist:

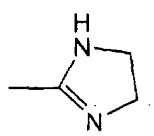

oder

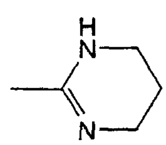

(ii) $H_2N-CH_2-CHO$, um eine Verbindung der Formel (I) zu erhalten, wobei B ist:

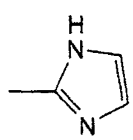

(iii) $H_2N-CN$, um eine Verbindung der Formel (I) zu erhalten, wobei B ist:

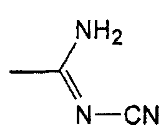

(iv) $H_2N-OR_6$, um eine Verbindung der Formel (I) zu erhalten, wobei B ist:

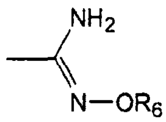

(v) $H_2N\text{-}NH_2$, um eine Verbindung der Formel (I) zu erhalten, wobei B ist:

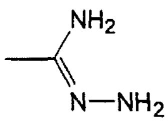

(vi) $HNR_4R_5$, um eine Verbindung der Formel (I) zu erhalten, wobei B ist:

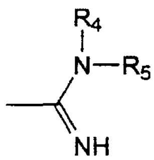

und anschließend gegebenenfalls mit $H_2NR_3$, um eine Verbindung der Formel (I) zu erhalten, wobei B ist:

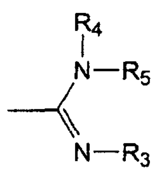

(vii) Bernsteinsäureanhydrid, um eine Verbindung der Formel (I) zu erhalten, wobei B ist -C≡N;
(viii) Wasser in einem alkalischen Medium, um eine Verbindung der Formel (I) zu erhalten, wobei B $-CO\text{-}NR_9R_{10}$ ist, wobei $R_9$ und $R_{10}$ beide Wasserstoffatome sind;
(ix) $HNR_9R_{10}$, um eine Verbindung der Formel (I) zu erhalten, wobei B ist:

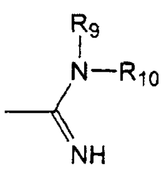

und dann mit Wasser in einem alkalischen Medium, um eine Verbindung der Formel (I) zu erhalten, wobei B $-CO\text{-}NR_9R_{10}$ ist, wobei $R_9$ und $R_{10}$ wie in Anspruch 1 definiert sind;
oder:

(b) wenn B nicht

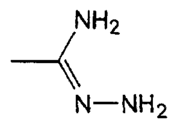

ist, Reagieren einer Verbindung der Formel:

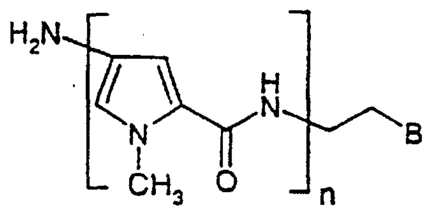

(IV)

mit einer Verbindung der Formel:

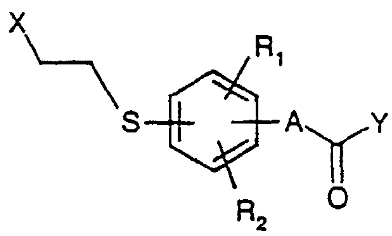

(III)

worin n, B, $R_1$, $R_2$, X, Y und A wie oben definiert sind;

um die entsprechende Verbindung der Formel (I) zu erhalten, und, wenn gewünscht, Umwandeln der Verbindung der Formel (I) in ein pharmazeutisch annehmbares Salz davon.

**6.** Verfahren gemäß Anspruch 5, wobei bei den Verbindungen der Formel (III) Y Hydroxy oder eine Gruppe ausgewählt aus Chlor, 2,4,5-Trichlorphenoxy, 2,4-Dinitrophenoxy, Succinimido-N-oxy und Imidazolyl ist.

**7.** Pharmazeutische Zusammensetzung umfassend einen oder mehrere pharmazeutisch annehmbare Träger und/oder Verdünner und, als wirksamen Inhaltsstoff, eine Verbindung gemäß Anspruch 1.

**8.** Verbindung gemäß Anspruch 1 zur Verwendung in einem Behandlungsverfahren einer Therapie eines menschlichen oder tierischen Körpers.

**9.** Verbindung gemäß Anspruch 8 zur Verwendung als Antitumormittel.

**10.** Verwendung einer Verbindung gemäß Anspruch 1 zur Herstellung eines Medikaments zur Verwendung als Antitumormittel.

## Revendications

**1.** Composé qui est un dérivé sulfuré de la distamycine de formule :

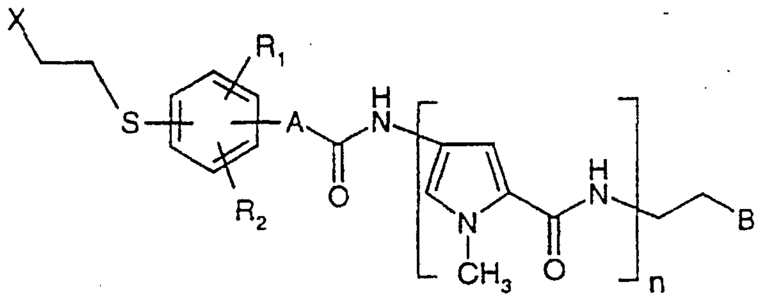

(I)

dans laquelle :

n est 2, 3 ou 4 ;

A est une liaison, un groupe alkylène en $C_1$-$C_4$ ou alcénylène en $C_2$-$C_4$ ;

$R_1$ et $R_2$, qui sont identiques ou différents, sont choisis parmi hydrogène, alkyle en $C_1$-$C_4$ éventuellement substitué par un ou plusieurs atome(s) de fluor, et alkoxy en $C_1$-$C_4$ ;

X est un atome d'halogène ;

B est choisi parmi :

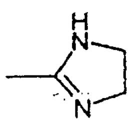 , 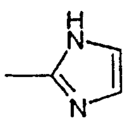 , 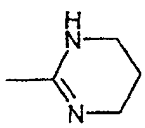 , 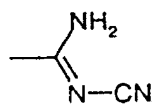 , 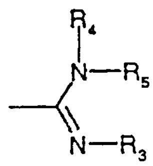 , 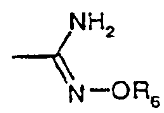 ,

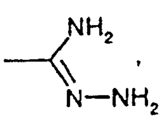 , —CN , 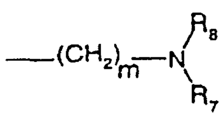 , 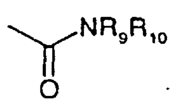 et 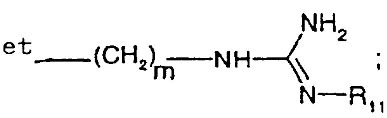 ;

où $R_3$, $R_4$, $R_5$, $R_6$, $R_7$, $R_8$, $R_9$ et $R_{10}$, qui sont identiques ou différents, sont choisis parmi hydrogène ou alkyle en $C_1$-$C_4$ ; $R_{11}$ est hydrogène, alkyle en $C_1$-$C_4$ ou hydroxy, et m est 0, 1 ou 2 ; ou sel pharmaceutiquement acceptable de celui-ci.

**2.** Composé selon la revendication 1, pour lequel $R_3$, $R_4$, $R_5$, $R_6$, $R_7$, $R_8$, $R_9$, $R_{10}$ et $R_{11}$ sont, indépendamment l'un de l'autre, hydrogène, méthyle ou éthyle.

**3.** Composé selon la revendication 1 ou 2, pour lequel n est 3 ;

A est une liaison ou vinylène ;

$R_1$ et $R_2$ qui sont identiques ou différents, sont choisis parmi hydrogène, méthyle, méthoxy et trifluorométhyle ;

X est chloro ou bromo ;

B est choisi parmi :

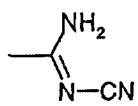 , 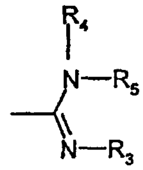 , 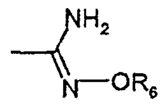 , —CN ,

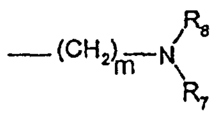 , 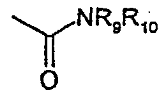 et 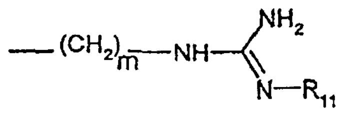

où $R_3$, $R_4$, $R_5$, $R_7$, $R_8$, $R_9$, $R_{10}$ et $R_{11}$, qui sont identiques ou différents, sont choisis parmi hydrogène et méthyle ; $R_6$ est hydrogène ; et m est 0 ou 1 ; ou sel pharmaceutiquement acceptable de celui-ci.

**4.** Composé selon la revendication 1 choisi dans le groupe constitué par :

3-[1-méthyl-4[1-méthyl-4[1-méthyl-4[4-(2-chloroéthylthio]phényl-1-carboxamido]pyrrole-2-carboxamido]pyrrole-2-carboxamido]pyrrole-2-carboxamido]propionamidine ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propion-N-méthylamidine ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propion-N,N'-diméthylamidine ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propion-N,N',N'-triméthylamidine ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propion-N-cyanamidine ;
3-[1-méthyl-4[1-méthyl-4[1-méthyl-4[4-(2-chloroéthylthio)phényl-1-carboxamido]pyrrole-2-carboxamido]pyrrole-2-carboxamido] pyrrole-2-carboxamido]propionamidoxime ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propionamide ;
3-[1-méthyl-4[1-méthyl-4[1-méthyl-4[4-(2-chloroéthylthio)phényl-1-carboxamido]pyrrole-2-carboxamido]pyrrole-2-carboxamido] pyrrole-2-carboxamido]propion-N-méthylamide ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propionitrile ;
2-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]éthylguanidine ;
3-[1-méthyl-4[1-méthyl-4[1-méthyl-4[4-(2-chloroéthylthio)phényl-1-carboxamido]pyrrole-2-carboxamido]pyrrole-2-carboxamido]pyrrole-2-carboxamido]propion-N,N-diméthylamine ;
3-[1-méthyl-4[1-méthyl-4[1-méthyl-4[4- (2-bromoéthylthio)phényl-1-carboxamido]pyrrole-2-carboxamido]pyrrole-2-carboxamido]pyrrole-2-carboxamido]propionamidine ;
3-[1-méthyl-4[1-méthyl-4[1-méthyl-4[3-méthyl-4-(2-chloroéthylthio)phényl-1-carboxamido]pyrrole-2-carboxamido]pyrrole-2-carboxamido]pyrrole-2-carboxamido]propionamidine ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propionamidine ;
3-[1-méthyl-4[1-méthyl-4[1-méthyl-4[4-(2-bromoéthylthio)phényl-1-carboxamido]pyrrole-2-carboxamido]pyrrole-2-carboxamido]pyrrole-2-carboxamido]propion-N-méthylamidine ;
3-(1-bromoéthylthio)phényl-1-carboxamido]pyrrole-2-carboxamido]pyrrole-2-carboxamido]pyrrole-2-carboxamido]propionamidoxime ;
2-[1-méthyl-4[1-méthyl-4[1-méthyl-4[4-(2-bromoéthylthio)phényl-1-carboxamido]pyrrole-2-carboxamido]pyrrole-2-carboxamido]pyrrole-2-carboxamido]éthylguanidine ;
3-[1-méthyl-4[1-méthyl-4[1-méthyl-4[3-méthyl-4-(2-chloroéthylthio]phényl-1-carboxamido]pyrrole-2-carboxamido]pyrrole-2-carboxamido]pyrrole-2-carboxamido]propion-N,N'-diméthylamidine ;
3-[1-méthyl-4[1-méthyl-4[1-méthyl-4[3-méthyl-4-(2-chloroéthylthio)phényl-1-carboxamido]pyrrole-2-carboxamido]pyrrole-2-carboxamido]pyrrole-2-carboxamido]propionamidoxime ;

3-[1-méthyl-4[1-méthyl-4[1-méthyl-4[3-méthyl-4-(2-chloroéthylthio)phényl-1-carboxamido]pyrrole-2-carboxamido]pyrrole-2-carboxamido]pyrrole-2-carboxamido]propionamide ;
3-[1-méthyl-4[1-méthyl-4[1-méthyl-4[3-méthyl-4-(2-chloroéthylthio)phényl-1-carboxamido]pyrrole-2-carboxamido]pyrrole-2-carboxamido]pyrrole-2-carboxamido]propionitrile ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido] pyrrole-2-carboxamido]propion-N-méthylamidine ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propion-N,N' -diméthylamidine ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propion-N-cyanamidine ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propionamidoxime ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propionamide ;
3-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]propionitrile ;
2-[1-méthyl-4[1-méthyl-4[1-méthyl-4[4-(2-chloroéthylthio)cinnamoyl-1-carboxamido]pyrrole-2-carboxamido] pyrrole-2-carboxamido]pyrrole-2-carboxamido]éthylguanidine ;

et les sels pharmaceutiquement acceptables de ceux-ci.

**5.** Procédé pour préparer un composé de formule (I) tel que défini dans la revendication 1, lequel procédé comprend les étapes consistant à :

(a) quand B est autre que

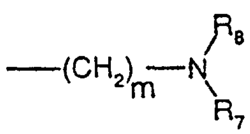

et

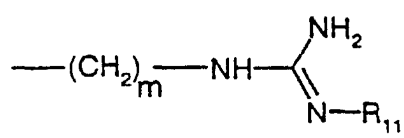

faire réagir un composé de formule :

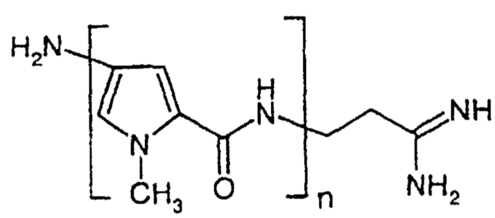

(II)

avec un composé de formule :

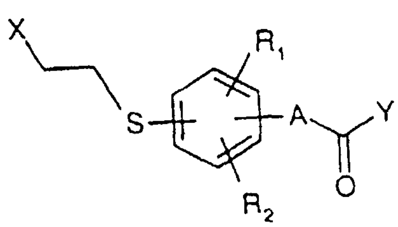

(III)

dans lesquelles n, $R_1$, $R_2$, X et A sont tels que définis dans la revendication 1, et Y est hydroxy ou un groupe partant approprié ;

afin d'obtenir un composé de formule :

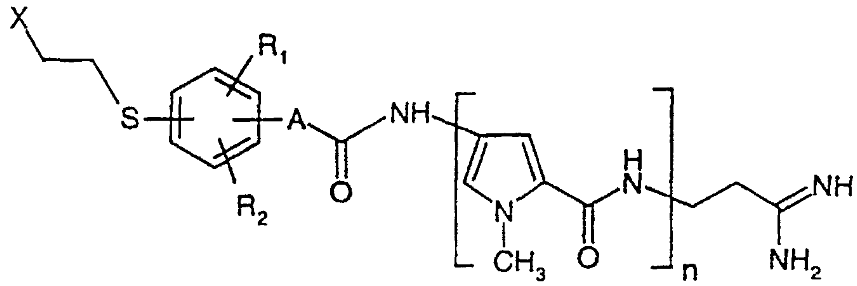

(Ia)

et, puis, éventuellement faire réagir un composé de formule (Ia) avec :

(i) $H_2N-(CH_2)_r-NH_2$, où r est 2 ou 3, de façon à obtenir un composé de formule (I) ayant B égal à :

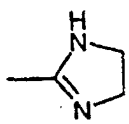

ou

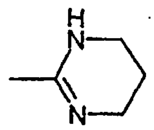

(ii) $H_2N-CH_2-CHO$, pour obtenir un composé de formule (I) ayant B égal à :

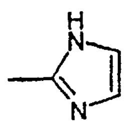

(iii) $H_2N-CN$, pour obtenir un composé de formule (I) ayant B égal à :

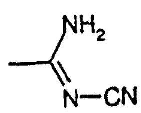

(iv) $H_2N-OR_6$, pour obtenir un composé de formule (I) ayant B égal à :

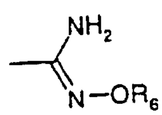

(v) $H_2N-NH_2$, pour obtenir un composé de formule (I) ayant B égal à :

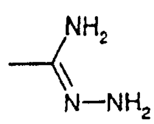

(vi) $HNR_4R_5$, pour obtenir un composé de formule (I) ayant B égal à :

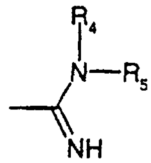

et puis éventuellement avec $H_2NR_3$, pour obtenir un composé de formule (I) ayant B égal à :

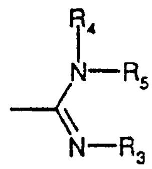

(vii) l'anhydride succinique, pour obtenir un composé de formule (I) ayant B égal à -C≡N ;
(viii) l'eau dans un milieu alcalin, pour obtenir un composé de formule (I) ayant B égal à $-CO-NR_9R_{10}$ où $R_9$ et $R_{10}$ sont tous les deux des atomes d'hydrogène ;
(ix) $HNR_9R_{10}$, pour obtenir un composé de formule (I) ayant B égal à :

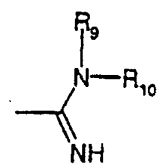

puis avec de l'eau dans un milieu alcalin, pour obtenir un composé de formule (I) ayant B égal à $-CO-NR_9R_{10}$, où $R_9$ et $R_{10}$ sont tels que définis dans la revendication 1 ; ou :
(b) quand B est autre que

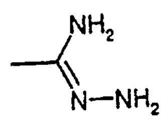

faire réagir un composé de formule :

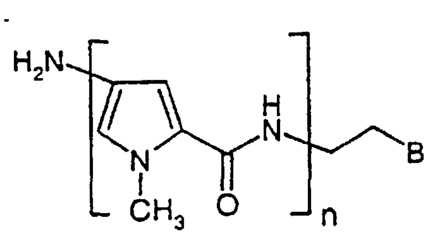

(IV)

avec un composé de formule :

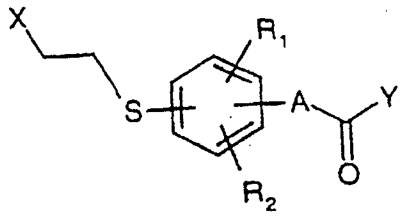

(III)

dans lesquelles n, B, $R_1$, $R_2$, X, Y et A sont tels que définis ci-dessus ; pour obtenir le composé correspondant de formule (I) et, si souhaité, convertir le composé de formule (I) en sel pharmaceutiquement acceptable de celui-ci.

**6.** Procédé selon la revendication 5 pour lequel, dans les composés de formule (III), Y est hydroxy ou un groupe choisi parmi chloro, 2,4,5-trichlorophénoxy, 2,4-dinitrophénoxy, succinimido-N-oxy et imidazolyle.

**7.** Composition pharmaceutique comprenant un ou plusieurs véhicule(s) et/ou diluant(s) pharmaceutiquement acceptable(s) et, en tant que principe actif, un composé tel que défini dans la revendication 1.

**8.** Composé tel que défini dans la revendication 1 pour une utilisation dans une méthode de traitement du corps humain ou animal par thérapie.

**9.** Composé tel que défini dans la revendication 8 pour une utilisation en tant qu'agent antitumoral.

**10.** Utilisation d'un composé tel que défini dans la revendication 1 dans la fabrication d'un médicament pour une utilisation en tant qu'agent antitumoral.